# EUROPEAN PATENT APPLICATION

(11) **EP 4 527 826 A1**
(43) Date of publication of application: **26.03.2025**
(21) Application number: 23894928.3
(22) Date of filing: 16.11.2023
(51) Int. Cl.: C07C 7/12, C07C 11/16

(54) **METHOD FOR RECOVERING CONJUGATED DIENE-BASED MONOMER**

(30) Priority: 23.11.2022 KR 20220158562
(71) Applicant: LG Chem, Ltd., Yeongdeungpo-gu Seoul 07336 (KR)
(72) Inventor: CHO, Yong Heon, Daejeon 34122 (KR); KO, Jun Seok, Daejeon 34122 (KR); OH, Suk Yung, Daejeon 34122 (KR); BAEK, Yong Jin, Daejeon 34122 (KR); CHOI, Sung Jun, Daejeon 34122 (KR)
(74) Representative: Goddar, Heinz J.
(86) International application number: PCT/KR2023/018452
(87) International publication number: WO 2024/111999

(57) **Abstract**

Provided is a method for recovering a conjugated diene-based monomer including: supplying a feed stream including a conjugated diene-based monomer to a condensation part and performing condensation to obtain a stream including a condensed conjugated diene-based monomer and a stream including an uncondensed conjugated diene-based monomer; recovering conjugated diene-based monomer from stream including condensed conjugated diene-based monomer, and supplying stream including an uncondensed conjugated diene-based monomer to a first absorption tower and bringing into contact with a sorbent to obtain an absorbed solution in which conjugated diene-based monomer is absorbed into sorbent; and supplying absorbed solution to a stripping column to separate absorbed solution into sorbent and conjugated diene-based monomer, circulating a lower discharge stream from the stripping column including the separated sorbent to the first absorption tower, and circulating an upper discharge stream from the stripping column including separated conjugated diene-based monomer to condensation part.

## Description

### [Technical Field]

### CROSS-REFERENCE TO RELATED APPLICATIONS

The present application claims the benefit of priorities to Korean Patent Application No. 10-2022-0158562, filed on November 23, 2022, and Korean Patent Application No. 10-2023-0158834, filed on November 16, 2023, the entire contents of which are incorporated herein as a part of the specification.

### Technical Field

The present invention relates to a method for recovering a conjugated diene-based monomer, and more particularly, to a method of adding a sorbent to remove uncondensed gas such as nitrogen and effectively separating and recovering the sorbent and the conjugated diene-based monomer through a cooling part.

### [BACKGROUND]

In general, during a process of preparing a nitrile butadiene rubber latex (NBL), in a polymer aqueous solution obtained after a polymerization reaction of a nitrile-based monomer and a conjugated diene-based monomer, the conjugated diene-based monomer and the nitrile-based monomer which do not react in the polymerization reaction as well as a copolymer latex may be included.

Herein, the nitrile-based monomer included in the polymer aqueous solution is separated and recovered through a compression process and an absorption tower. As such, a remainder after separating the nitrile-based monomer includes the conjugated diene-based monomer, and conventionally, the remainder (mixed gas) after separating the nitrile-based monomer is supplied to a condenser and condensed, and then recovered in a liquid state. However, due to uncondensed gas such as nitrogen which is added or introduced during the process, vapor pressure of the mixed gas is increased, and a large amount of the conjugated diene-based monomer is uncondensed and flared with nitrogen. When the conjugated diene-based monomer is flared as such, environmental problems due to greenhouse gases and total volatile organic compounds (tVOC) produced in this process and the loss cost of conjugated diene-based monomer are significant.

Therefore, a process which may separate the conjugated diene-based monomer to be recovered from uncondensed gas and increase a recovery rate of the conjugated diene-based monomer should be introduced.

### [SUMMARY]

### [Technical Problem]

In order to solve the problems mentioned in the Background Art, an object of the present invention is to provide a method for recovering a conjugated diene-based monomer, which may effectively separate the conjugated diene-based monomer from uncondensed gas such as nitrogen and increase a recovery rate of the conjugated diene-based monomer.

### [Technical Solution]

In one general aspect, a method for recovering a conjugated diene-based monomer includes: supplying a feed stream including a conjugated diene-based monomer to a condensation part and performing condensation to obtain a stream including a condensed conjugated diene-based monomer and a stream including an uncondensed conjugated diene-based monomer; recovering a conjugated diene-based monomer from the stream including a condensed conjugated diene-based monomer, and supplying the stream including an uncondensed conjugated diene-based monomer to a first absorption tower and bringing it into contact with a sorbent to obtain an absorbed solution in which the conjugated diene-based monomer is absorbed into the sorbent; and supplying the absorbed solution to a stripping column to separate the absorbed solution into the sorbent and the conjugated diene-based monomer, circulating a lower discharge stream from the stripping column including the separated sorbent to the first absorption tower, and circulating an upper discharge stream from the stripping column including the separated conjugated diene-based monomer to the condensation part.

### [Advantageous Effects]

According to the method for recovering a conjugated diene-based monomer of the present invention, unabsorbed gas including nitrogen which is not absorbed into a sorbent may be separated from a conjugated diene-based monomer to recover the conjugated diene-based monomer. Specifically, the conjugated diene-based monomer is selectively absorbed into the sorbent to obtain an absorbed solution, thereby recovering the conjugated diene-based monomer from the absorbed solution. Thus, an amount of loss of the conjugated diene-based monomer which is separated and flared with the unabsorbed gas may be minimized.

Meanwhile, a temperature in an upper portion of a stripping column is directly adjusted by a cooling part provided in a top stage of the stripping column, thereby preventing a fouling occurrence in the stripping column. Besides, the temperature in the upper portion of the stripping column is adjusted by the cooling part as such, thereby easily performing separation of the sorbent and the conjugated diene-based monomer in the stripping column, and also increasing a recovery rate of the conjugated diene-based monomer and the sorbent.

### [BRIEF DESCRIPTION OF THE DRAWINGS]

FIG. 1 is a process flow chart according to a method for recovering a conjugated diene-based monomer according to an exemplary embodiment of the present invention.
FIG. 2 is a process flow chart according to a method for recovering a conjugated diene-based monomer according to Comparative Example 1 of the present invention.
FIG. 3 is a process flow chart according to a method for recovering a conjugated diene-based monomer according to Comparative Example 2 of the present invention.

### [DETAILED DESCRIPTION]

The terms and words used in the description and claims of the present invention are not to be construed limitedly as having general or dictionary meanings but are to be construed as having meanings and concepts meeting the technical ideas of the present invention, based on a principle that the inventors are able to appropriately define the concepts of terms in order to describe their own inventions in the best mode.

In the present invention, the term "stream" may refer to a fluid flow in a process, or may refer to a fluid itself flowing in a pipe. Specifically, the "stream" may refer to both a fluid itself flowing in a pipe connecting each device and a fluid flow. In addition, the fluid may include any one or more components of gas, liquid, and solid.

Meanwhile, in the devices such as an absorption tower, a stripping column, a distillation column, and a recovery tower in the present invention, unless otherwise particularly stated, a "lower portion" of the device refers to a point at a height of 95% to 100% downward from the top of the device, specifically the lowest part (column bottom). Likewise, unless otherwise particularly stated, an "upper portion" of the device refers to, a point at a height of 0% to 5% downward from the top of the device, specifically the top (column top).

Meanwhile, in the devices such as an absorption tower, a stripping column, a distillation column, and a recovery tower in the present invention, unless otherwise particularly stated, "pressure" of the device may refer to upper pressure of the device.

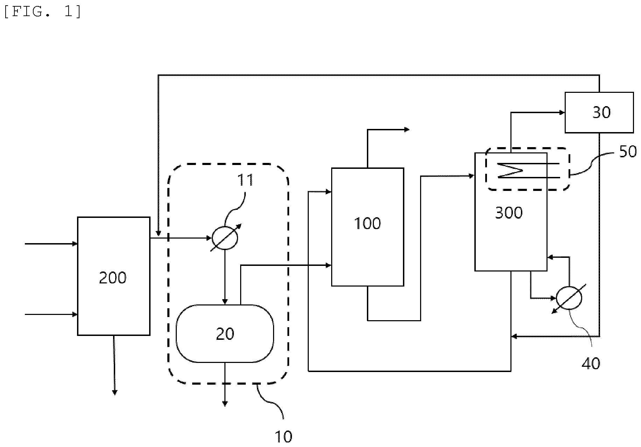

Hereinafter, the present invention will be described in more detail with reference to FIG. 1 for better understanding of the present invention.

A method for recovering a conjugated diene-based monomer according to an exemplary embodiment of the present invention may include: supplying a feed stream including a conjugated diene-based monomer to a condensation part 10 and performing condensation to obtain a stream including a condensed conjugated diene-based monomer and a stream including an uncondensed conjugated diene-based monomer; recovering a conjugated diene-based monomer from the stream including a condensed conjugated diene-based monomer, and supplying the stream including an uncondensed conjugated diene-based monomer to a first absorption tower 100 and bringing it into contact with a sorbent to obtain an absorbed solution in which the conjugated diene-based monomer is absorbed into the sorbent; and supplying the absorbed solution to a stripping column 300 to separate the absorbed solution into the sorbent and the conjugated diene-based monomer, circulating a lower discharge stream from the stripping column including the separated sorbent to the first absorption tower 100, and circulating an upper discharge stream from the stripping column including the separated conjugated diene-based monomer to the condensation part 10.

First, the method for recovering a conjugated diene-based monomer according to an exemplary embodiment of the present invention may include: supplying a feed stream including a conjugated diene-based monomer to a condensation part 10 and performing condensation to obtain a stream including a condensed conjugated diene-based monomer and a stream including an uncondensed conjugated diene-based monomer.

Herein, the feed stream including a conjugated diene-based monomer may be derived from a stream separated from a copolymer latex in a polymerization process in which a reactant including a nitrile-based monomer and a conjugated diene-based monomer is polymerized to obtain the copolymer latex. More specifically, the polymerization process may be performed by including a polymerization reaction step and a vacuum stripping step. The polymerization reaction step may be performed by supplying a reactant including a nitrile-based monomer and a conjugated diene-based monomer to a reactor and polymerizing the reactant, and a polymer aqueous solution may be obtained from the polymerization reaction as such. Herein, water may be further supplied to the reactor. The polymer aqueous solution may include water, a nitrile-based monomer, a conjugated diene-based monomer, and a copolymer latex. Herein, the nitrile-based monomer and the conjugated diene-based monomer may be an unreacted monomer which is not polymerized in the polymerization reaction.

In the present invention, the nitrile-based monomer may include one or more selected from the group consisting of acrylonitrile, methacrylonitrile, fumaronitrile, α-chloronitrile, and α-cyanoethylacrylonitrile, but is not limited thereto. As a specific example, the nitrile-based monomer may be acrylonitrile.

Meanwhile, the conjugated diene-based monomer may include one or more selected from the group consisting of 1,3-butadiene, 1,4-butadiene, 2,3-dimethyl-1,3-butadiene, 2-ethyl-1,3-butadiene, 1,3-pentadiene, piperylene, 3-butyl-1,3-octadiene, 2-phenyl-1,3-butadiene, and isoprene, but is not limited thereto. As a specific example, the conjugated diene-based monomer may be 1,3-butadiene or 1,4-butadiene.

In order to obtain the copolymer latex included in the polymer aqueous solution, a vacuum stripping step may be performed. By-products, the nitrile-based monomer, and the conjugated diene-based monomer included in the polymer aqueous solution may be separated from the copolymer latex by vacuuming and heating performed in the vacuum stripping step. Therefore, the copolymer latex may be finally obtained, and the copolymer latex may be, for example, a nitrile-butadiene rubber latex (NBL).

The stream separated from the copolymer latex in the polymerization process may further undergo a compression process. Specifically, the stream separated from the copolymer latex may be compressed and condensed in the compression process. Thus, water and the nitrile-based monomer are separated and a remainder separated from water and the nitrile-based monomer may be supplied to a second absorption tower 200. Herein, water may be treated as wastewater, and the nitrile-based monomer may be separately recovered and reused as a reactant in the polymerization process. Meanwhile, a remainder separated from the water and the nitrile-based monomer may include the conjugated diene-based monomer.

According to the present invention, the remainder separated from the water and the nitrile-based monomer after the compression process may be supplied to a lower portion of the second absorption tower 200 to further separate a part of the nitrile-based monomer. Herein, the part of the nitrile-based monomer separated in the second absorption tower 200 may be a nitrile-based monomer which is not separated in the compression process.

Specifically, a solvent is supplied to the upper portion of the second absorption tower 200 and the nitrile-based monomer is dissolved in the solvent to obtain a nitrile-based monomer aqueous solution. Herein, the solvent may include water. The nitrile-based monomer may be recovered from the nitrile-based monomer aqueous solution and reused as a reactant in the polymerization reaction step described above. Meanwhile, a stream including the remainder separated from the nitrile-based monomer aqueous solution in the second absorption tower 200 may be supplied to a condensation part 10 as a feed stream of the present invention. Herein, the feed stream may include nitrogen, oxygen, and C4-based impurities (i-butane, n-butane, 1-butene, i-butene, trans/cis-2- butene, 1,2-butadiene), in addition to the conjugated diene-based monomer.

The condensation part 10 may include a condenser 11 and a flash tank 20. Specifically, a feed stream including a conjugated diene-based monomer may be supplied to the condenser 11 and condensed, thereby obtaining a condensation product. The condensation product may include a conjugated diene-based monomer condensed by the condenser 11 and an uncondensed conjugated diene-based monomer. Herein, a temperature at which condensation is performed in the condenser 11 may be 10°C or higher or 12°C or higher and 20°C or lower or 18°C or lower.

Subsequently, the condensation product may be supplied to a flash tank 20 and separated into a liquid phase and a gas phase. Specifically, the condensation product may be separated into a stream including a condensed conjugated diene-based monomer and a stream including an uncondensed conjugated diene-based monomer in the flash tank 20. More specifically, the stream including a condensed conjugated diene-based monomer may be obtained as a liquid phase and the stream including an uncondensed conjugated diene-based monomer may be obtained as a gas phase. Herein, the conjugated diene-based monomer may be recovered from the stream including a condensed conjugated diene-based monomer and reused as a reactant in the polymerization process described above.

Meanwhile, the stream including an uncondensed conjugated diene-based monomer may further include nitrogen, oxygen, and C4-based impurities, in addition to the conjugated diene-based monomer. Herein, the conjugated diene-based monomer may be a monomer which is uncondensed in the condenser 11 and not recovered in the flash tank 20.

Meanwhile, when the stream including an uncondensed conjugated diene-based monomer is flared as before, environmental problems occur by greenhouse gases and total volatile organic compounds (tVOC) produced in this process. In addition, the loss cost of the conjugated diene-based monomer included in the stream including an uncondensed conjugated diene-based monomer were excessively increased. Therefore, the present invention may increase a recovery rate of the conjugated diene-based monomer and also solve environmental problems by flare, by separately separating and recovering the conjugated diene-based monomer included in the stream including an uncondensed conjugated diene-based monomer.

According to the present invention, a content of the conjugated diene-based monomer included in the stream including an uncondensed conjugated diene-based monomer may be 80 wt% or more, 84 wt% or more, or 88 wt% or more and 95 wt% or less, 94 wt% or less, or 92 wt% or less. When the content of the conjugated diene-based monomer included in the stream including an uncondensed conjugated diene-based monomer is within the range, a load of the first absorption tower 100 described later may be decreased to maximize absorption efficiency in the first absorption tower 100 compared with the same facility size. Therefore, it may be preferred in terms of economic feasibility such as process equipment and device costs.

The method for recovering a conjugated diene-based monomer according to an exemplary embodiment of the present invention may include: supplying the stream including an uncondensed conjugated diene-based monomer to a first absorption tower 100 and bringing it into contact with a sorbent to obtain an absorbed solution in which the conjugated diene-based monomer is absorbed into the sorbent.

Specifically, the conjugated diene-based monomer included in the stream including an uncondensed conjugated diene-based monomer may be absorbed into the sorbent, thereby obtaining the absorbed solution. Meanwhile, unabsorbed gas including components which are not dissolved in the sorbent may be separated from the absorbed solution, and the unabsorbed gas may include C4-based impurities, nitrogen, and oxygen. Therefore, the lower discharge stream from the first absorption tower including an absorbed solution and the upper discharge stream from the first absorption tower including unabsorbed gas may be separated in the first absorption tower 100. Herein, the upper discharge stream from the first absorption tower may be separately flared. By separating the conjugated diene-based monomer by absorbing it in the sorbent in the first absorption tower 100, an amount of loss of the conjugated diene-based monomer may be minimized as compared with a conventional method in which the stream including an uncondensed conjugated diene-based monomer is flared.

Meanwhile, when the unabsorbed gas including nitrogen and oxygen is not separated and removed, and recirculated to the condenser 11 with the conjugated diene-based monomer, an amount of the conjugated diene-based monomer uncondensed in the condenser 11 may be excessively increased due to the increased vapor pressure, so that a yield of the condensed conjugated diene-based monomer may be decreased, and thus, it may be preferred to separate the unabsorbed gas in the first absorption tower 100.

Operation pressure of the first absorption tower 100 for this may be 2 to 5 kgf/cm²(g), specifically 2.5 to 3.5 kgf/cm²(g). The operation pressure of the first absorption tower 100 may be gauge pressure. By operating the pressure of the first absorption tower 100 within the range, the conjugated diene-based monomer may be effectively absorbed into the sorbent, and it may be easy to separate the unabsorbed gas and the absorbed solution in the first absorption tower. Therefore, the content of the conjugated diene-based monomer included in upper discharge stream from the first absorption tower may be minimized.

In the present invention, the sorbent may be a hydrocarbon compound having 6 to 30 carbon atoms, and specifically, may include kerosene or diesel oil (gas oil). More specifically, the kerosene and diesel oil are the results of distillation of crude oil, and may be obtained in a stage lower than naphtha and higher than heavy oil (fuel oil) in a crude oil distillation column. A boiling point of the kerosene may be 140°C or higher, 150°C or higher, or 200°C or higher and 300°C or lower, 270°C or lower, or 250°C or lower, and a boiling point of the diesel oil may be 130°C or higher, 150°C or higher, or 200°C or higher and 380°C or lower, 360°C or lower, or 350°C or lower. When the kerosene or the diesel oil is used as the sorbent of the present invention, the conjugated diene-based monomer included in the stream including an uncondensed conjugated diene-based monomer may be effectively absorbed into the sorbent.

Meanwhile, a mass flow rate of the sorbent added to the first absorption tower 100 may be 5 times or more, 7 times or more, or 9 times or more and 15 times or less, 13 times or less, or 11 times or less the mass flow rate of the stream including an uncondensed conjugated diene-based monomer supplied to the first absorption tower 100. When the flow rate of the sorbent added to the first absorption tower 100 is multiples within the range as compared with the flow rate of the stream including an uncondensed conjugated diene-based monomer, the conjugated diene-based monomer is easily absorbed by the sorbent, thereby minimizing an amount of loss of the conjugated diene-based monomer which is flared and lost with the unabsorbed gas through the upper discharge stream from the first absorption tower.

According to the present invention, the content of the conjugated diene-based monomer included in the absorbed solution may be 1 wt% or more, 5 wt% or more, or 7 wt% or more and 20 wt% or less, 15 wt% or less, or 10 wt% or less. In the case in which content of the conjugated diene-based monomer included in the absorbed solution, that is, the lower discharge stream from the first absorption tower is within the range, an appropriate amount of the conjugated diene-based monomer is absorbed into the sorbent, and an excessive rise in temperature by heat discharged as absorption proceeds in the first absorption tower 100 may be prevented. In addition, solubility of the conjugated diene-based monomer may be increased to further maximize absorption efficiency in the first absorption tower 100.

The method for recovering a conjugated diene-based monomer according to an exemplary embodiment of the present invention may include: supplying the absorbed solution to a stripping column 300 to separate the absorbed solution into the sorbent and the conjugated diene-based monomer, circulating a lower discharge stream from the stripping column including the separated sorbent to the first absorption tower 100, and circulating an upper discharge stream from the stripping column including the separated conjugated diene-based monomer to the condensation part 10.

Specifically, the lower discharge stream from the first absorption tower including the absorbed solution may be supplied to the stripping column 300 to separate it into a lower fraction of the stripping column 300 including the sorbent and an upper fraction of the stripping column 300 including the conjugated diene-based monomer. The lower fraction of the stripping column 300 may be circulated to the first absorption tower 100 as a lower discharge stream from the stripping column, and the upper fraction of the stripping column 300 may be circulated to the condensation part 10 as an upper discharge stream from the stripping column.

More specifically, heat is supplied to the stripping column 300 by a reboiler 40 provided downstream of the stripping column 300, and the conjugated diene-based monomer vaporized by the process may be moved to an upper portion of the stripping column 300. Temperature in the upper portion of the stripping column 300 is raised by the conjugated diene-based monomer moved to the upper portion of the stripping column 300 to cause fouling.

Therefore, in the present invention, the stripping column 300 may include a cooling part 50 in the top stage. The stripping column 300 is a column formed of a plurality of stages, and the top stage may be a top stage where a column top is positioned among the plurality of stages. Specifically, the cooling part 50 may be provided with a cooling coil, and the temperature at a height of 0 to 20% of the stripping column 300, more specifically, the temperature in the upper portion of the stripping column 300 may be lowered by the cooling coil. Furthermore, the sorbent which is vaporized with the conjugated diene-based monomer by the reboiler 40 may be cooled in the cooling part 50 in the top stage of the stripping column to separate the cooled sorbent and the conjugated diene-based monomer.

The temperature at a height of 0 to 20% based on the top 0% of the stripping column 300 may be controlled to 10°C or more, 12°C or more, or 14°C or more and 30°C or less, 28°C or less, or 20°C or less by the cooling part 50. Herein, the top of the stripping column 300 may refer to a column top. For example, when the cooling part 50 is provided in the top stage of the stripping column 300, the temperature of the top of the stripping column 300 is the lowest and temperature may rise from the top to the lower portion of the stripping column 300. Therefore, the temperature in an area at a height of 0% to 20% of the stripping column 300 may belong to a temperature range of 10°C to 30°C by the cooling part 50.

Specifically, it may be preferred that the temperature at the height of 0 to 20% of the stripping column 300 is adjusted to the range, in terms of cooling the vaporized sorbent to separate it with the conjugated diene-based monomer, and it may also reduce a risk of fouling due to a temperature rise in the stripping column 300. Herein, the temperature at the height of 0 to 20% of the stripping column 300 may be controlled by the cooling part 50 described above, and the temperature of the cooling coil provided in the cooling part 50 is adjusted to be lower than the temperature of the stripping column 300 targeted in the present invention, thereby controlling the temperature at the height of 0 to 20% of the stripping column 300 to 10°C to 30°C.

More specifically, when the temperature at the height of 0 to 20% of the stripping column 300 is lower than 10°C, the conjugated diene-based monomer may be condensed with the sorbent due to the excessively low temperature and exist inside the stripping column 300. In addition, in order to satisfy the temperature conditions, a new cooling source is needed instead of an existing chilled water used in the cooling part, and thus, it may not be preferred from an economic point of view such as additional process equipment and device costs. Meanwhile, the temperature at the height of 0 to 20% of the stripping column 300 is higher than 30°C, it may be difficult to condensate the sorbent through the cooling part, and thus, it may be difficult to effectively separate the sorbent and the conjugated diene-based monomer in the stripping column 300. In addition, it may be difficult to prevent a fouling occurrence in the stripping column 300.

Meanwhile, the operation pressure of the stripping column 300 may be -0.9 kgf/cm²(g) or more, -0.8 kgf/cm²(g) or more, or -0.7 kgf/cm²(g) or more and -0.1 kgf/cm²(g) or less, -0.2 kgf/cm²(g) or less, or -0.4 kgf/cm²(g) or less. Herein, the operation pressure of the stripping column 300 is a gauge pressure, which means a value obtained by subtracting atmospheric pressure from absolute pressure. Since a boiling point difference between materials to be separated in the stripping column 300 is increased by operating the pressure of the stripping column 300 within the range, the separation efficiency of the stripping column 300 may be increased. Furthermore, the pressure is lowered from the first absorption tower 100 to the stripping column 300, thereby better performing separation of the conjugated diene-based monomer in the stripping column 300. Therefore, separation of the conjugated diene-based monomer and the sorbent in the stripping column 300 may be effectively performed.

By the control of the operation pressure and the temperature of the stripping column 300 as such, the amount of the lost sorbent may be minimized to reuse the recovered sorbent in the first absorption tower 100. Therefore, the amount of the fresh sorbent supplied to the first absorption tower 100 may be minimized.

Meanwhile, before the upper discharge stream from the stripping column is circulated to the condensation part 10, the upper discharge stream from the stripping column is supplied to a vacuum compression part 30 and compressed, thereby supplying the stream including the sorbent to the first absorption tower 100 and circulating the stream including the compressed conjugated diene-based monomer to the condensation part 10.

Specifically, the process performed in the vacuum compression part 30 may be performed by including a first compression step and a second compression step. First, the upper discharge stream from the stripping column may be introduced to the first compression step. More specifically, in the first compression step, the upper discharge stream from the stripping column may be supplied to a vacuum pump and compressed, supplied to an aftercooler and cooled, and then separated into a liquid stream including the sorbent and a gaseous stream including the conjugated diene-based monomer. Herein, the vacuum pump may be the same device as the compressor, but pressures in the former/latter stages may be different. Specifically, the vacuum pump may be operated at the pressure of the former stage of vacuum.

Next, the gaseous stream separated through the first compression step may be introduced to the second compression step. More specifically, in the second compression step, the gaseous stream may be supplied to the compressor and compressed, and supplied to the aftercooler and cooled. Thus, the sorbent cooled in the aftercooler in the second compression step may be separated from the compressed conjugated diene-based monomer. Therefore, a stream including the sorbent cooled in the aftercooler in the second compression step and a stream including the compressed conjugated diene-based monomer may be obtained. Herein, the sorbent cooled in the aftercooler in the second compression step may be a sorbent which is not separated in the first compression step.

Meanwhile, the compressed conjugated diene-based monomer is in a gaseous state, and may be in a state pressurized by the vacuum compression part 30 for decreasing differential pressure when supplying the stream including the compressed conjugated diene-based monomer to the condensation part 10. Finally, the gaseous stream separated in the first compression step and the stream including the sorbent cooled in the aftercooler in the second compression step are streams including the sorbent, and include a trace amount of the sorbent which is not separated in the stripping column 300 in the former stage and may be circulated to the first absorption tower 100. Specifically, the stream including the sorbent is mixed with the lower discharge stream from the stripping column to form a mixed stream, and the mixed stream may be supplied to the first absorption tower 100. Meanwhile, the stream including the compressed conjugated diene-based monomer may be circulated to the condensation part 10.

Hereinafter, the present invention will be described in more detail by the examples. However, the following examples are provided for illustrating the present invention, and it is apparent to a person skilled in the art that various modifications and alterations may be made without departing from the scope and spirit of the present invention, and the scope of the present invention is not limited thereto.

### Examples

### Example 1

According to the process flow illustrated in FIG. 1, a process of recovering a conjugated diene-based monomer was simulated, using an Aspen Plus simulator available from Aspen.

Specifically, a feed stream including a conjugated diene-based monomer, nitrogen, oxygen, and C4-based impurities was supplied to a condensation part 10 at a flow rate of 2000 kg/hr. More specifically, the feed stream was supplied to the condenser 11 and condensed to obtain a condensation product, which was supplied to a flash tank 20 to obtain a stream including a condensed conjugated diene-based monomer and a stream including an uncondensed conjugated diene-based monomer. The conjugated diene-based monomer was recovered from the stream including a condensed conjugated diene-based monomer. At this time, condensation performed in the condenser 11 was performed at a temperature of 20°C. Meanwhile, the content of the conjugated diene-based monomer included in the stream including an uncondensed conjugated diene-based monomer was 90 wt%.

The stream including an uncondensed conjugated diene-based monomer was supplied to a first absorption tower 100 and brought into contact with kerosene as a sorbent to obtain an absorbed solution in which the conjugated diene-based monomer was absorbed into the sorbent. The upper discharge stream from the first absorption tower including nitrogen and oxygen as the components which were not absorbed into the sorbent was flared, and the lower discharge stream from the first absorption tower including the absorbed solution was supplied to the first stage from the top of the stripping column 300. At this time, operation pressure of the first absorption tower 100 was 3 kgf/cm²(g). In addition, the mass flow rate of the sorbent introduced to the first absorption tower 100 was 10 times the mass flow rate of the stream including an uncondensed conjugated diene-based monomer supplied to the first absorption tower 100.

Subsequently, the absorbed solution was separated into the sorbent and the conjugated diene-based monomer in the stripping column 300, thereby obtaining the lower discharge stream from the stripping column including the separated sorbent and the upper discharge stream from the stripping column including the separated conjugated diene-based monomer. At this time, the temperature in the upper portion of the stripping column 300 was 10°C by a cooling part 50 provided in the top stage of the stripping column 300, and the temperature in the lower portion of the stripping column 300 was 144°C by a reboiler 40 provided downstream of the stripping column 300. In addition, the operation pressure of the stripping column 300 was **-0.8** kgf/cm²(g). The lower discharge stream from the stripping column was circulated to the first absorption tower 100, and the upper discharge stream from the stripping column was supplied to a vacuum compression part 30. At this time, the upper discharge stream from the stripping column was discharged at a temperature of 10°C.

Next, a stream including the sorbent and a stream including the compressed conjugated diene-based monomer were obtained by a process performed in the vacuum compression part 30. The stream including the sorbent was mixed with the lower discharge stream from the stripping column to obtain a mixed stream, and the mixed stream was supplied to the first absorption tower 100. The stream including the compressed conjugated diene-based monomer was mixed with the feed stream and supplied to the condensation part 10.

As a result, the recovery rate of the conjugated diene-based monomer was 100 wt% and the loss rate of the sorbent was 0.007 wt%.

### Example 2

In Example 2, a conjugated diene-based monomer was recovered by the same process flow as in Example 1, except that the cooling part was not provided in the top stage of the stripping column.

As a result, the temperature in the upper portion of the stripping column was 40°C, the upper discharge stream from the stripping column was discharged at a temperature of 40°C, and the recovery rate of the conjugated diene-based monomer and the loss rate of the sorbent are shown in the following Table 1.

### Example 3

In Example 3, a conjugated diene-based monomer was recovered by the same process flow as in Example 1, except that the temperature in the upper portion of the stripping column was controlled to 15°C by the cooling part.

### Example 4

In Example 4, a conjugated diene-based monomer was recovered by the same process flow as in Example 1, except that the temperature in the upper portion of the stripping column was controlled to 35°C by the cooling part.

### Comparative Examples

### Comparative Example 1

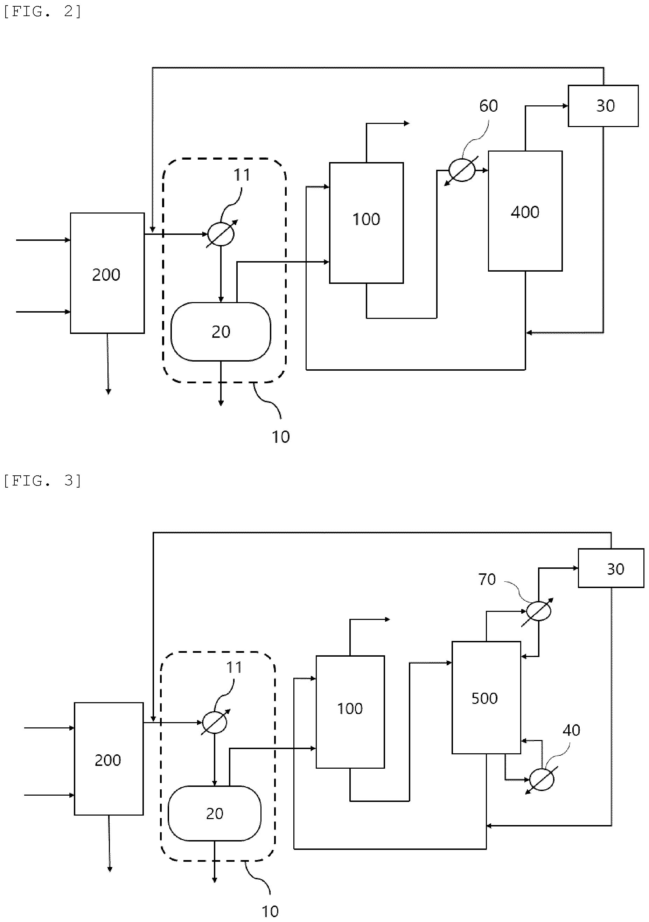

According to the process flow illustrated in FIG. 2, a process of recovering a conjugated diene-based monomer was simulated, using an Aspen Plus simulator available from Aspen.

In comparative Example 1, a flash tank 400 was provided instead of the stripping column, and a conjugated diene-based monomer was recovered by the same process flow as in Example 1, except that the temperature in the upper portion and the temperature in the lower portion of the flash tank, and the temperature at which the conjugated diene-based monomer is discharged was 40°C.

Specifically, a feed stream including a conjugated diene-based monomer, nitrogen, oxygen, and C4-based impurities was supplied to a condensation part 10 at a flow rate of 2000 kg/hr. The feed stream was condensed in the condensation part 10 to obtain a stream including a condensed conjugated diene-based monomer and a stream including an uncondensed conjugated diene-based monomer. At this time, the condensation was performed at a temperature of 20°C. Meanwhile, the content of the conjugated diene-based monomer included in the stream including an uncondensed conjugated diene-based monomer was 90 wt%.

The stream including the uncondensed conjugated diene-based monomer was supplied to the first absorption tower 100 and brought into contact with kerosene as a sorbent to obtain an upper discharge stream from the first absorption tower including unabsorbed gas and a lower discharge stream from the first absorption tower including an absorbed solution. At this time, operation pressure of the first absorption tower 100 was 3 kgf/cm²(g). In addition, the mass flow rate of the sorbent introduced to the first absorption tower 100 was 10 times the mass flow rate of the stream including an uncondensed conjugated diene-based monomer supplied to the first absorption tower 100.

Subsequently, the lower discharge stream from the first absorption tower was supplied to the flash tank 400 via a heater 60. At this time, the temperature of the heater was 40°C. In the flash tank 400, the absorbed solution included in the lower discharge stream from the first absorption tower was separated into the sorbent and the conjugated diene-based monomer, thereby obtaining the lower discharge stream from the flash tank including the separated sorbent and the upper discharge stream from the flash tank including the separated conjugated diene-based monomer. At this time, the temperature of the flash tank 400 was 40°C, and the operation pressure was -0.8 kgf/cm²(g). The lower discharge stream from the flash tank was circulated to the first absorption tower 100, and the upper discharge stream from the flash tank was supplied to a vacuum compression part 30. At this time, the upper discharge stream from the flash tank was discharged at a temperature of 40 °C.

Next, a stream including the sorbent and a stream including the compressed conjugated diene-based monomer were obtained by a process performed in the vacuum compression part 30. The stream including the sorbent was mixed with the lower discharge stream from the flash tank to obtain a mixed stream, and the mixed stream was supplied to the first absorption tower 100. The stream including the compressed conjugated diene-based monomer was mixed with the feed stream and supplied to the condensation part 10.

As a result, the recovery rate of the conjugated diene-based monomer was 100 wt% and the loss rate of the sorbent was 0.013 wt%.

### Comparative Example 2

According to the process flow illustrated in FIG. 3, a process of recovering a conjugated diene-based monomer was simulated, using an Aspen Plus simulator available from Aspen.

In Comparative Example 1, a conjugated diene-based monomer was recovered by the same process flow as in Example 1, except that a distillation column 500 was provided instead of the stripping column.

Specifically, a feed stream including a conjugated diene-based monomer, nitrogen, oxygen, and C4-based impurities was supplied to a condensation part 10 at a flow rate of 2000 kg/hr. The feed stream was condensed in the condensation part 10 to obtain a stream including a condensed conjugated diene-based monomer and a stream including an uncondensed conjugated diene-based monomer. At this time, the condensation was performed at a temperature of 20°C. Meanwhile, the content of the conjugated diene-based monomer included in the stream including an uncondensed conjugated diene-based monomer was 90 wt%.

The stream including the uncondensed conjugated diene-based monomer was supplied to the first absorption tower 100 and brought into contact with kerosene as a sorbent to obtain an upper discharge stream from the first absorption tower including unabsorbed gas and a lower discharge stream from the first absorption tower including an absorbed solution. At this time, operation pressure of the first absorption tower 100 was 3 kgf/cm²(g). In addition, the mass flow rate of the sorbent introduced to the first absorption tower 100 was 10 times the mass flow rate of the stream including an uncondensed conjugated diene-based monomer supplied to the first absorption tower 100.

Subsequently, the lower discharge stream from the first absorption tower was supplied to the distillation column 500, the absorbed solution included in the lower discharge stream from the first absorption tower was separated into the sorbent and the conjugated diene-based monomer, thereby obtaining the lower discharge stream from the distillation column including the separated sorbent and the upper discharge stream from the distillation column including the separated conjugated diene-based monomer. At this time, the temperature in the upper portion of the distillation column 500 was 40°C and the temperature in the lower portion was 144°C. In addition, the operation pressure of the distillation column 500 was -0.8 kgf/cm²(g). The lower discharge stream from the distillation column was circulated to the first absorption tower 100, and a part of the upper discharge stream from the distillation column was supplied to the vacuum compression part 30 and the rest thereof was refluxed to the distillation column 500 via a condenser 70. At this time, the upper discharge stream from the distillation column through the condenser 70 was discharged at a temperature of 10°C.

Next, a stream including the sorbent and a stream including the compressed conjugated diene-based monomer were obtained by a process performed in the vacuum compression part 30. The stream including the sorbent was mixed with the lower discharge stream from the distillation column to obtain a mixed stream, and the mixed stream was supplied to the first absorption tower 100. The stream including the compressed conjugated diene-based monomer was mixed with the feed stream and circulated to the condensation part 10.

The operation conditions, the recovery rate of the conjugated diene-based monomer, the loss rate of the sorbent, and a fouling occurrence of the device in the examples and the comparative examples are shown in the following Table 1.

The recovery rate of the conjugated diene-based monomer is a percentage of a value obtained by subtracting the mass flow rate of the conjugated diene-based monomer included in the upper discharge stream from the first absorption tower from the mass flow rate of the conjugated diene-based monomer included in the stream including the uncondensed conjugated diene-based monomer, based on the mass flow rate of the conjugated diene-based monomer included in the stream including the uncondensed conjugated diene-based monomer.

The loss rate of the sorbent is a percentage of the mass flow rate of the sorbent included in the stream including the condensed conjugated diene-based monomer obtained from the upper discharge stream from the first absorption tower and the vacuum compression part to the mass flow rate of the sorbent included in the mixed stream supplied to the first absorption tower.

**[Table 1]**

| | | Example 1 | Example 2 | Example 3 | Example 4 | Comparative Example 1 | | Comparative Example 2 | |
|---|---|---|---|---|---|---|---|---|---|
| First absorption tower | Pressure (kgf/cm²(g)) | 3 | 3 | 3 | 3 | 3 | | 3 | |
| | Sorbent circulation flow rate ⁴⁾ (times) | 10 | 10 | 10 | 10 | 10 | | 10 | |
| | Temperature at which sorbent was added (°C) | 10 | 10 | 10 | 10 | 10 | | 10 | |
| Stripping column | Pressure (kgf/cm²(g)) | -0.8 | -0.8 | -0.8 | -0.8 | Flash tank ¹⁾ | -0.8 | Distilla tion column ²⁾ | -0.8 |
| | Temperature in upper portion (°C) | 10 | 40 | 15 | 35 | | 40 | | 40 |
| | Temperature in lower portion (°C) | 144 | 144 | 144 | 144 | | 40 | | 144 |
| | Terrperature at which conjugated diene-based monomer was discharged ³⁾ (°C) | 10 | 40 | 15 | 35 | | 40 | | 10 |
| Recovery rate of conjugated diene-based monomer (wt%) | | 100 | 100 | 100 | 100 | 99.6 | | 100 | |
| Loss rate of sorbent (wt%) | | 0.007 | 0.013 | 0.011 | 0.013 | 0.013 | | 0.007 | |
| Fouling occurrence | | | | × | ∘ | × | × | × | ∘ |
| 1) In Comparative Example 1, the flash tank was provided instead of the stripping column, and the pressure for the flash tank, the temperature in the upper portion, the temperature in the lower portion, and the temperature at which the conjugated diene-based monomer was discharged are listed in order. | | | | | | | | | |
| 2) In Comparative Example 2, the distillation column was provided instead of the stripping column, and the pressure for the distillation column, the temperature in the upper portion, the temperature in the lower portion, and the temperature at which the conjugated diene-based monomer was discharged are listed in order. | | | | | | | | | |
| 3) The temperature at which the conjugated diene-based monomer was discharged represents the temperature of the upper discharge stream from the stripping column, the upper discharge stream from the flash tank, or the upper discharge stream from the distillation column, including the conjugated diene-based monomer supplied from the stripping column, the flash tank, or the distillation column to the vacuum compression part. | | | | | | | | | |
| 4) The sorbent circulation flow rate represents the mass flow rate of the sorbent added to the first absorption tower to the mass flow rate of the stream including the uncondensed conjugated diene-based monomer supplied to the first absorption tower. | | | | | | | | | |

Referring to Table 1, it was confirmed that the examples provided with the stripping column had an excellent recovery rate of the conjugated diene-based monomer of 100 wt%. Furthermore, it was confirmed that Examples 1, 3, and 4 in which the cooling part was provided in the top stage of the stripping column to control the temperature in the upper portion of the stripping column had no fouling occurrence as compared with Comparative Example 2 and had a good loss rate of the sorbent as compared with Comparative Example 1. In particular, in Example 1 which had a big difference between the temperature in the upper portion and the temperature in the lower portion of the stripping column, it was confirmed that the separation efficiency of the stripping column was increased, specifically, the sorbent was condensed well by the low temperature in the upper portion of the stripping column, and the loss rate of the sorbent was the lowest among the examples.

However, in Comparative Example 1 provided with the flash tank instead of the stripping column, it was confirmed that the loss of the sorbent was increased as separation of the conjugated diene-based monomer and the sorbent proceeded, depending on the pressure and the temperature of the flash tank, and the loss rate of the sorbent was 1.8 times that of Example 1.

Meanwhile, in Comparative Example 2 provided with the distillation column instead of the stripping column, since the distillation column was operated under vacuum, the conjugated diene-based monomer was hardly condensed by the condenser upstream of the distillation column under the conditions. Therefore, since the conjugated diene-based monomer was hardly condensed by the condenser upstream of the distillation column and only the vaporized sorbent was condensed, the reflux flow rate in the upper portion of the distillation column was small. In this case, since the reflux flow rate in the upper portion of the distillation column was small, the cooling effect in the upper portion of the distillation column was insignificant, so that it was difficult to control the temperature in the upper portion of the distillation column and fouling occurred. At this time, a fouling occurrence was indirectly confirmed from the temperature in the distillation column.

## Claims

1. A method for recovering a conjugated diene-based monomer, the method comprising:
supplying a feed stream including a conjugated diene-based monomer to a condensation part and performing condensation to obtain a stream including a condensed conjugated diene-based monomer and a stream including an uncondensed conjugated diene-based monomer;
recovering the conjugated diene-based monomer from the stream including the condensed conjugated diene-based monomer, and
supplying the stream including the uncondensed conjugated diene-based monomer to a first absorption tower and bringing the uncondensed conjugated diene-based monomer into contact with a sorbent to obtain an absorbed solution in which the conjugated diene-based monomer is absorbed into the sorbent; and
supplying the absorbed solution to a stripping column to separate the absorbed solution into the sorbent and the conjugated diene-based monomer, circulating a lower discharge stream from the stripping column including the separated sorbent to the first absorption tower, and circulating an upper discharge stream from the stripping column including the separated conjugated diene-based monomer to the condensation part.

2. The method for recovering a conjugated diene-based monomer of claim 1,
wherein the feed stream including a conjugated diene-based monomer is derived from a stream separated from a copolymer latex in a polymerization process of obtaining the copolymer latex by polymerizing a reactant including a nitrile-based monomer and the conjugated diene-based monomer.

3. The method for recovering a conjugated diene-based monomer of claim 2,
wherein the feed stream including a conjugated diene-based monomer is obtained by supplying the stream separated from the copolymer latex to a second absorption tower and separating the nitrile-based monomer.

4. The method for recovering a conjugated diene-based monomer of claim 1,
wherein a content of the conjugated diene-based monomer included in the stream including an uncondensed conjugated diene-based monomer is 80 to 95 wt%.

5. The method for recovering a conjugated diene-based monomer of claim 1,
wherein the sorbent is a hydrocarbon compound having 6 to 30 carbon atoms.

6. The method for recovering a conjugated diene-based monomer of claim 1,
wherein a mass flow rate of the sorbent added to the first absorption tower is 5 to 15 times a mass flow rate of the stream including an uncondensed conjugated diene-based monomer supplied to the first absorption tower.

7. The method for recovering a conjugated diene-based monomer of claim 1,
wherein a content of the conjugated diene-based monomer included in the absorbed solution is 5 to 20 wt%.

8. The method for recovering a conjugated diene-based monomer of claim 1,
wherein the stripping column includes a cooling part in a top stage.

9. The method for recovering a conjugated diene-based monomer of claim 8,
wherein a temperature at a height of 0 to 20% based on the top 0% of the stripping column is controlled to 10°C to 30°C by the cooling part.

10. The method for recovering a conjugated diene-based monomer of claim 1, further comprising:
before circulating the upper discharge stream from the stripping column to the condensation part,
supplying the upper discharge stream from the stripping column to a vacuum compression part and performing compression, thereby supplying a stream including the sorbent to the first absorption tower and circulating a stream including the compressed conjugated diene-based monomer to the condensation part.

11. The method for recovering a conjugated diene-based monomer of claim 1,
wherein an operation pressure of the first absorption tower is 2 to 5 kgf/cm²(g).

12. The method for recovering a conjugated diene-based monomer of claim 1,
wherein an operation pressure of the stripping column is -0.9 to -0.1 kgf/cm²(g).
